# EUROPEAN PATENT APPLICATION

(11) **EP 2 913 060 A1**
(43) Date of publication of application: **02.09.2015**
(21) Application number: 14305295.9
(22) Date of filing: 28.02.2014
(51) Int. Cl.: A61K 38/17, A61P 17/00

(54) **INHIBITION OF THE FRACTALKINE-RECEPTOR INTERACTION FOR THE TREATMENT OF ATOPIC DERMATITIS**

(71) Applicant: UNIVERSITE DE NICE SOPHIA-ANTIPOLIS, 06103 Nice Cédex 02 (FR); Centre National de la Recherche Scientifique, 75016 Paris (FR)
(72) Inventor: Julia, Valérie, 06410 Biot (FR); Dombrowicz, David, 59000 Lille (FR); Glaichenhaus, Nicolas, 06100 Nice (FR)
(74) Representative: Rançon, Xavier Lucien Abel

(57) **Abstract**

The invention relates to the treatment of atopic dermatitis through inhibition of the expression of fractalkine or of its receptor CX3CR1, or through inhibition of their interaction.

## Description

The present invention relates to the treatment of atopic diseases through the inhibition of the interaction between fractalkine (CX3CL1) and its receptor (CX3CR1).

Atopic dermatitis (AD) is a common, chronic inflammatory dermatosis frequently occuring in individuals with a personal or family history of atopic diseases. AD pathophysiology is complex and results from skin barrier dysfunction and a dysregulated immune response, influenced by genetic and environmental factors (Guttman-Yassky *et al.*, 2011a; Guttman-Yassky *et al.,* 2011b). Most patients with AD have increased serum IgE levels, with specific IgE directed against allergens or microbial proteins such as *Staphylococcus aureus* (Leung *et al.*, 2004). Lesions in AD are characterized by increased epidermal thickness and a dermal inflammatory cell infiltrate, consisting of mast cells, eosinophils and T lymphocytes. In acute AD lesions, a preferential recruitment of Th2 cells occurs, whereas in the chronic lesions a Th1 profile is predominant (Grewe *et al.*, 1998), while allergic asthma or allergic rhinitis are more exclusively Th2-dominated diseases.

Chemokines and their receptors play a key role in leukocyte recruitment to inflamed skin (Schall and Proudfoot, 2011). Eotaxins 1, 2 and 3 (CCL11, 24, 26) bind to CCR3 and attract eosinophils, and CCL26 appears to be particularly involved in AD (Kagami *et al.,* 2003; Owczarek *et al.,* 2011). CCL27 together with CCR10 and CCR4 expression insure T cell skin domiciliation (Homey *et al.,* 2002; Reiss *et al.,* 2001). More recently, CCR8 and CCL8 have been demonstrated to direct Th2 cell recruitment into allergen-inflamed skin and draining lymph nodes in a murine model of AD (Islam *et al.,* 2011).

Besides chemoattraction, chemokine - chemokine receptor interactions also regulate other functions. The inventors have recently demonstrated that CX3CR1, the receptor for CX3CL1 (fractalkine -CX3-), identified also as a receptor for CCL26 (Nakayama *et al.*, 2010) in humans, controls the development of allergic asthma by providing a survival signal to the CD4⁺ effector T lymphocytes in the inflammatory airways (Julia, 2012; Mionnet *et al.*, 2010). Mionnet *et al.* (2010) suggest that blocking CX3CR1 signaling pathway may represent a promising therapy for treating asthma.

In AD patients, CX3CL1 is upregulated in both endothelial cells and skin lesions and serum CX3CL1 levels are positively associated with disease severity (Echigo *et al.,* 2004). Echigo *et al.* (2004) has also reported that the endothelial CX3CL1 (fractalkine) expression and frequency of infiltrating CX3CR1⁺ cells in the lesional skin were increased in patients with psoriasis. Another study reported that, while CX3CR1 mRNA expression is consistently upregulated in AD skin, CX3CL1 mRNA levels are only increased in some patients with a significant correlation to the disease severity (Nakayama *et al.*, 2010), a result likely to explain the earlier failure to detect CX3CL1 in skin lesions (Fraticelli *et al.*, 2001). Furthermore, two CX3CR1 single nucleotide polymorphisms have been associated with asthma and atopy in French-Canadian populations (Tremblay *et al.*, 2006) and German children (Depner *et al.*, 2007).

International Application WO 2005/030245 discloses the use of an inhibitor of the expression of fractalkine or of CX3CR1 or the use of a fractalkine/CX3CR1 antagonist for preparing a medicament for treating an atopic allergic disease. WO 2005/030245 further discloses experimental results in mouse suggesting that neutralization of CX3CL1 with an anti-CX3CL1 antibody results in decreased cell infiltration in the airways, in decreased eosinophilia and in decreased Th2 response to LACK (see Example 6 of WO 2005/030245).

Patent Application EP 1 806 145 discloses the use of an antibody or a CX3CR1 antagonist inhibiting the CX3CL1/CX3CR1 interaction for treating inflammatory diseases. EP 1 806 145 further discloses experimental results in mouse suggesting that neutralization of CX3CL1 with an anti-CX3CL1 antibody is useful for treating inflammatory bowel diseases.

The experimental results disclosed in WO 2005/030245 and Mionnet *et al.* (2010) relate to a disease affecting the lungs (asthma) while the experimental results disclosed in EP 1 806 145 relate to a disease affecting the bowels (inflammatory bowel disease). These references do not disclose any experimental result concerning the treatment of atopic dermatitis or using antagonist of CX3CR1. Further, the lung and the bowel are physiologically different organs from the skin. In addition, the inventors have shown in a mouse psoriasis model that inhibition of CX3CL1/CX3CR1 interaction using a CX3CR1 antagonist (CX3-AT peptide) is not useful for treating psoriasis even if endothelial CX3CL1 expression is augmented with increased infiltration of CX3CR1⁺ cells in the lesional skin of patients with psoriasis (as shown by Echigo *et al.*, 2004) and also contrary to what is stated (without any experimental results) in EP 1 806 145. Jung *et al.* (2000) have shown, in the case of contact dermatitis induced by oxazolone that CX3CR1 knockout mice developed the same skin lesions as wild type mice; demonstrating that CX3CR1 plays no roles in this pathology while it concerns the skin. Consequently, therapeutic strategies for treating asthma (affecting the lungs) or inflammatory bowel diseases (affecting the bowels) cannot be transposed to the treatment of other pathologies concerning other organs such as atopic dermatitis (affecting the skin).

The inventors have thus investigated the role and mechanism of action of fractalkine (CX3CL1) and its unique receptor (CX3CR1) in experimental models of AD and psoriasis. Only AD pathology and immune responses were profoundly decreased in CX3CR1-deficient mice as well as upon blocking CX3CL1/CX3CR1 interactions in wild type mice. CX3CR1 deficiency neither affected antigen presentation nor T cell proliferation *in vivo* upon skin sensitization, but CX3CR1 expression by both Th2 and Th1 cells was required to induce AD. Surprisingly, unlike in allergic asthma, where CX3CL1 and CX3CR1 regulate the pathology by controlling effector CD4⁺ T cell survival within inflamed tissues, adoptive transfer studies established CX3CR1 as a key regulator of CD4⁺ T cell retention in inflamed skin. Further, the inventors have unexpectedly found that CX3CL1/CX3CR1 controlled atopic dermatitis to an even greater extent than allergic asthma.

Accordingly, the present invention provides an inhibitor of the expression of fractalkine (CX3CL1) or an inhibitor of the expression of the receptor of fractalkine (CX3CR1) or an inhibitor of the CX3CL1/CX3CR1 interaction for use in the treatment of atopic dermatitis (AD).

Inhibitors of the expression of CX3CL1 or of CX3CR1 include for instance antisense oligonucleotides, or interfering RNAs, targeting the gene encoding CX3CL1 or the gene encoding CX3CR1 respectively.

By way of examples, small interfering RNA (siRNA) sequences directed to CX3CR1 were designed and validated by Liu *et al.* (2011). Small interfering RNA sequences, corresponding to bases 2290-2309 nt of the *Mus musculus* chemokine (C-X₃-C motif) ligand 1 (CX3CL1) gene (NCBI Accession No.: NM_009142), have been described to efficiently decreased CX3CL1 expression (Ren *et al.*, 2007).

An inhibitor of the CX3CL1/CX3CR1 interaction refers to a compound able to inhibit the activation of CX3CR1 by CX3CL1 (fractalkine), without being able to activate CX3CR1 on its own. This includes in particular compounds able to interact with CX3CL1, and to inhibit its binding with CX3CR1 or to inhibit the activation of CX3CR1 resulting from said binding, as well as compounds able to interact with CX3CR1, and to inhibit its binding with CX3CL1 or its activation resulting from said binding. In particular, inhibitors of the CX3CL1/CX3CR1 interaction encompass competitive and non-competitive antagonists of CX3CR1, preferably competitive antagonists of CX3CR1.

Inhibitors of the CX3CL1/CX3CR1 interaction include peptides, such as antagonist peptides of CX3CR1.

A preferred peptide is a peptide CX3-AT (see Inoue *et al.,* 2005; Mionnet *et al.,* 2010). CX3-AT is a CX3CR1 competitive antagonist having about 75 amino acids (70 to 80 amino acids) and a molecular weight of about 9.5 Da. The amino acid sequence of CX3-AT includes at least the minimal amino acid region of CX3CL1 required to allow the CX3CL1/CX3CR1 interaction.

The inventors have found in mice that the administration by topical route (i.e., local; applied to the surface of the skin) of a 95 amino acid peptide (SEQ ID NO: 31) comprising a CX3-AT peptide (SEQ ID NO: 32), a Bir A sequence and a His tag, and having a molecular weight of 11,149 Da, is more efficient to treat atopic dermatitis in comparison to systemic administration. This effect is unexpected because: i) based on physico/chemical testing, it has been defined in the prior art a 1000 Da threshold above which the penetration through the skin of molecules of higher molecular weight is impossible (Berard *et al.,* 2003) and ii) CX3CR1 is a chemokine receptor having a key role in the cell migration, and cell adhesion (Imai *et al.,* 1997; Fong *et al.,* 1998; Nanki *et al.,* 2002; McComb *et al.,* 2008; Ruitenberg *et al.*, 2008). Thus in view of the prior art it is unlikely to block cell migration or transmigration by administrating a compound by topical route, while it is likely by systemic administration.

Advantageously, the peptide CX3-AT can derive from CX3CL1 from mouse (such as the peptides CX3-AT of SEQ ID NO: 32 and 33), human (such as the peptide CX3-AT of SEQ ID NO: 34) or dog (such as the peptide CX3-AT of SEQ ID NO: 35).

Inhibitors of the CX3CL1/CX3CR1 interaction also include organic molecules. For example, International Application WO 02/076990 describes thiazolopyrimidine derivatives as CX3CR1 antagonists.

Inhibitors of the CX3CL1/CX3CR1 interaction also include peptidomimetics. A "peptidomimetic", refers to a molecule which is not a peptide, but which mimic aspects of its structures. Peptidomimetics can be designed, for example, by establishing the three dimensional structure of a peptide agent in the environment in which it is bound or will bind to CX3CR1. International Application WO 01/60406 discloses methods for obtaining such peptidomimetics.

Inhibitors of the CX3CL1/CX3CR1 interaction also include antibodies which can be polyclonal or monoclonal. The term "antibody" as used herein also encompasses functional fragments of antibodies, including fragments of chimeric, humanized, single chain antibodies or fragments thereof (*e.g*., Fv, Fab, Fab'and F(ab') 2 fragments). Suitable antibodies are those which are directed to CX3CL1 (fractalkine) or to CX3CR1. In preferred embodiment, said antibody is a monoclonal antibody, or fragment thereof.

Methods for preparing monoclonal antibodies are well known in the art. Monoclonal antibodies may be prepared by immunizing a mammal (*e.g*., a mouse, rat, camelid, human) with a purified CX3CR1, CX3CL1 (fractalkine) or portions thereof. The antibody-producing cells in the immunized mammal are isolated and fused with myeloma or heteromyeloma cells to produce hybrid cells (hybridoma). International Application WO 01/60406 discloses methods to produce antibodies suitable for use according to the invention.

Inhibitors of the CX3CL1/CX3CR1 interaction also include aptamers recognizing either CX3CR1 or CX3CL1 (fractalkine). Aptamers are single stranded nucleic acid molecules (DNA or RNA) able to bind to a target molecule.

Inhibitors of the CX3CL1/CX3CR1 interaction can be identified, for example, by screening a collection of candidate compounds for their ability to inhibit the activation of CX3CR1 in the presence of CX3CL1 (fractalkine). Methods for measuring the activation of G protein-coupled receptors that are known in themselves, and are for instance commonly used in high-throughput screening assays, can be used for evaluating the activation of CX3CR1. Examples of methods for assessing the activity of such inhibitors of the CX3CL1/CX3CR1 interaction are described in International Application WO 01/60406 and Patent Application EP 1 806 145.

Said inhibitors of the expression of CX3CL1 or of CX3CR1 or inhibitors of the CX3CL1/CX3CR1 interaction as defined above can be administered by themselves, or mixed with suitable carriers or excipient(s). They can be used systemically or locally. One can use any formulation suitable for systemic local administration. For local administration, one can use a solution, a suspension, a gel, an ointment, for administration on the skin (*i.e.,* for application to the surface of the skin). Local (*i.e.*, topical) administration is preferred in the case of peptide CX3-AT.

As used herein, the terms "treatment" or "treating" includes the administration of an inhibitor of the expression of CX3CL1 or of CX3CR1 or an inhibitor of the CX3CL1/CX3CR1 interaction as defined above to a subject who has a disorder, a symptom of disorder or a predisposition toward a disorder, with the purpose to cure, heal, alleviate, relieve, alter, remedy, ameliorate, improve or affect the disorder, the symptoms of the disorder, or the predisposition toward disorder.

The subject can be a human or an animal.

Animals include mammals, in particular dogs (*Canis lupus familiaris*).

In a particular embodiment of the present invention, said inhibitor of the CX3CL1/CX3CR1 interaction is a peptide CX3-AT derived from CX3CL1 from a dog (such as the peptide of SEQ ID NO: 35) and said subject to treat is a dog.

The present invention also provides a method for treating atopic dermatitis, comprising administering to a subject in need thereof an inhibitor of the expression of CX3CL1 or of CX3CR1 or an inhibitor of the CX3CL1/CX3CR1 interaction as defined above.

The present invention encompasses the peptides CX3-AT of SEQ ID NO: 34 and 35.

The present invention also encompasses a pharmaceutical composition comprising a peptide CX3-AT of SEQ ID NO: 34 or 35.

In addition to the preceding features, the invention further comprises other features which will emerge from the following description, which refers to non-limitative examples illustrating the present invention, as well as to the appended figures.

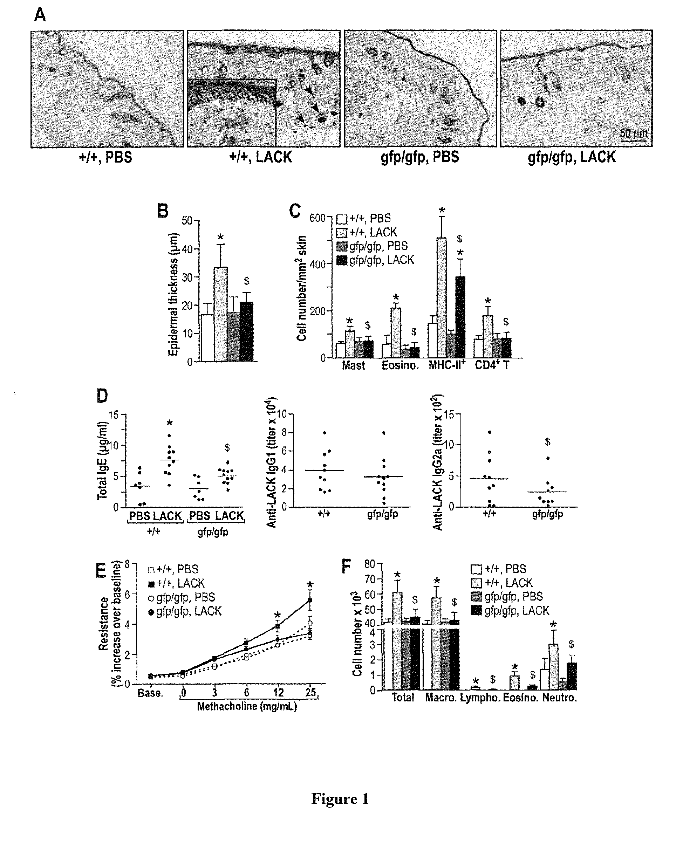

**Figure 1****.** Absence of atopic dermatitis and attenuation of associated humoral and lung inflammatory response in CX3CR1-deficient mice. AD was induced on abdominal skin in CX3CR1^{+/+} and CX3CR1^{gfp/gfp} mice by epicutaneous LACK sensitization for three 1-week periods, with a 2-week interval between applications. At day 49, sera were collected and animals were challenged by LACK nebulization. At day 50, AHR to increasing concentrations of methacholine was measured by invasive plethysmography. Then, mice were sacrificed and BAL fluid was collected and analyzed on cytospin preparations. Skin samples were collected at the site of sensitization. **A.** May Grünwald Giemsa staining of skin sections (original magnification X10 for the main panels and X63 for the inset). Black arrows indicate mast cells and white arrows in the inset indicate eosinophils. **B.** Epidermal thickness. **C.** Eosinophil, mast cell, MHC II⁺ and CD4⁺ T cell numbers in dermis. **D.** Immunoglobulin concentrations in serum. Total IgE (upper left panel), -LACK-specific IgG1 (bottom left panel) and LACK-specific IgG2a (bottom right panel) concentrations. **E.** Airway hyperreactivity to increasing methacholine concentrations. Resistance was evaluated by invasive plethysmography. **F.** Lung inflammatory response: total number of cells, macrophages, neutrophils, lymphocytes and eosinophils in BAL fluid. Data are expressed as mean ± SEM (*n*=6-10 animals per group). * Statistically different from PBS-treated mice (P < 0.05); $ statistically different from CX3CR1^{+/+} mice (P < 0.05).

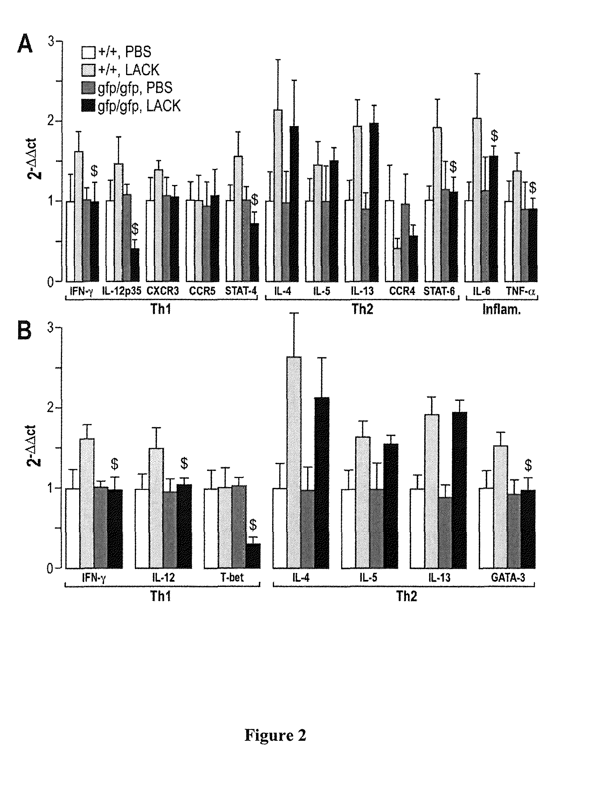

**Figure 2****.** Attenuation of associated skin and draining lymph node response associated to AD in CX3CR1-deficient mice. AD was induced on abdominal skin in CX3CR1^{+/+} and CX3CR1^{gfp/gfp} mice as described for Fig. 1. Abdominal skin collected at day 50 and steadystate mRNA expression was analyzed. **A.** Skin and **B.** Inguinal lymph node mRNA expression of cytokines, chemokines and receptors, transcription factors. Results are expressed as the mean ± SEM fold induction compared with the mean expression level in PBS-treated WT mice (n=6-10 animals per group). $ Statistically different from CX3CR1^{+/+} mice (P < 0.05).

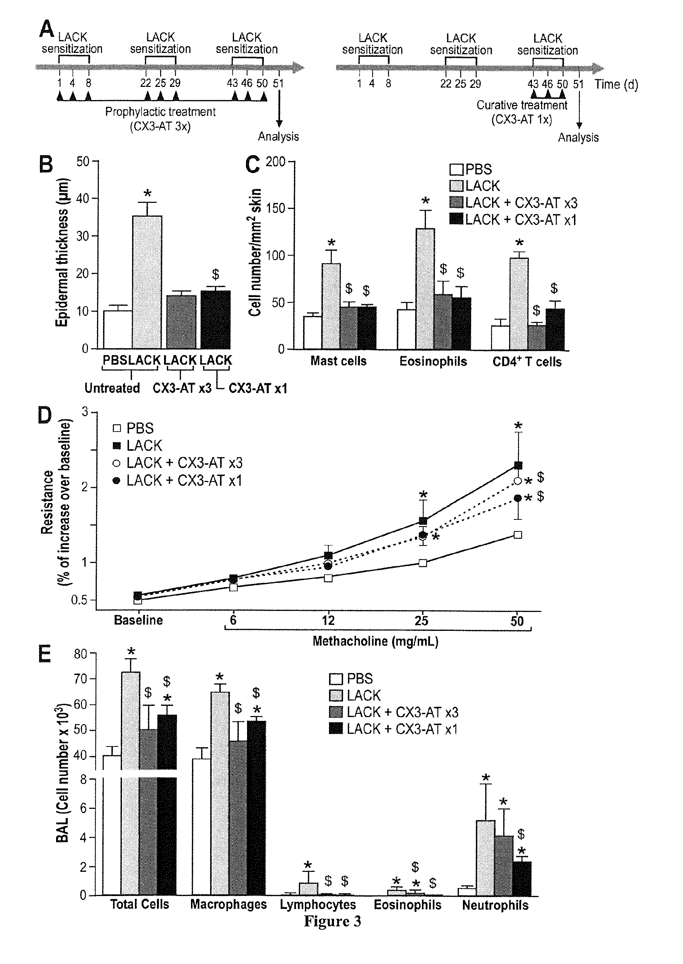

**Figure 3****.** Abrogation of AD features in wild-type mice treated with CX3-AT inhibitor. **A.** Timeline of CX3-AT administration during AD induction. **B.** Epidermal thickness. **C.** Eosinophil, mast cell, MHC II⁺ and CD4⁺ T cell numbers in dermis. **D.** Airway hyperreactivity to increasing methacholine concentrations. Resistance was evaluated by invasive plethysmography. **E.** Lung inflammatory response: total number of cells, macrophages, neutrophils, lymphocytes and eosinophils in BAL fluid. Data are expressed as mean ± SEM (*n*=6-10 animals per group). * Statistically different from PBS-treated mice (P < 0.05); $ statistically different from vehicle-treated mice (P < 0.05).

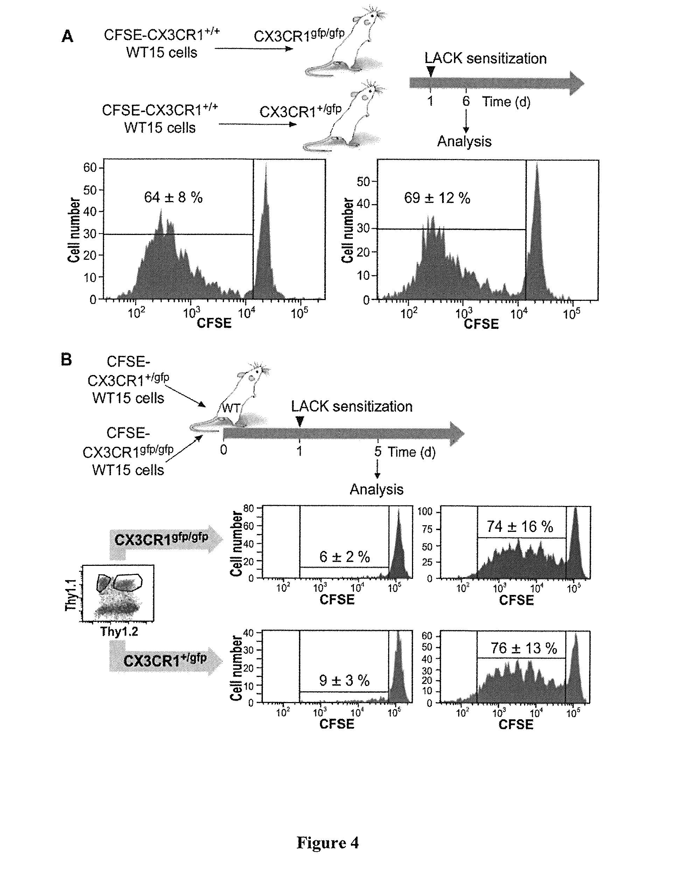

**Figure 4****.** CX3CR1 deficiency neither affects antigen presentation nor T cell proliferation. A. Lack of effect of CX3CR1 deficiency on antigen presentation. CSFE-labeled CX3CR1^{+/+} WT15 Thy1.1^{+/+} CD4⁺ T cells were injected *i.v*. into CX3CR1^{gfp/gfp} or CX3CR1^{+/gfp} mice one day before epicutaneous sensitization with LACK. Donor cells were analyzed by flow cytometry 5 days later in inguinal LN after gating onto CD4⁺ Thy1.1⁺ cells. Data show one representative flow cytometry profile of CFSE staining with numbers indicating frequencies of dividing donor cells ± SEM (n=6) **B.** Lack of effect of CX3CR1 deficiency on T cell proliferation. CSFE-labeled CX3CR1^{+/gfp} Thy1.1^{+/-} and CX3CR1^{gfp/gfp} Thy1.1^{+/+} WT15 CD4⁺ T cells were co-injected *i.v*. into WT Thy1.1^{-/-} Thy1.2^{+/+} mice one day before LACK or PBS epicutaneous sensitization. Donor cells were analyzed by flow cytometry 4 days later in inguinal LN after gating CD4⁺ Thy1.1⁺ cells. Data show a representative flow cytometry profile of CFSE staining with numbers indicating dividing CX3CR1^{+/+} and CX3CR1^{gfp/gfp} donor cells (n=5 mice in each group).

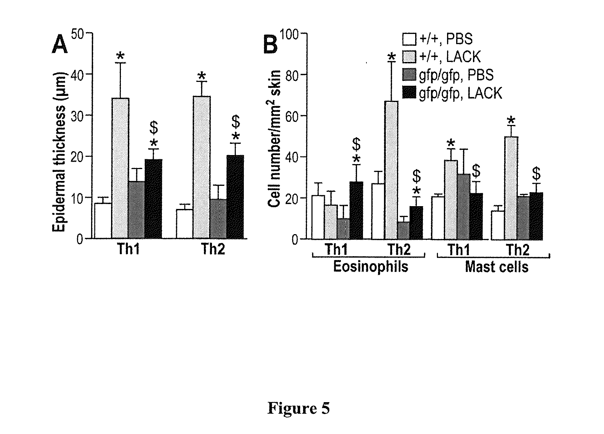

**Figure 5****.** CX3CR1 expression regulates both Th1- and Th2- induced skin inflammation. Wild-type mice were injected at day -1 with LACK-specific CX3CR1^{gfp/gfp} or CX3CR1^{+/gfp} Th1 or Th2 cells, sensitized for one single week with LACK or PBS at day 0 and further analyzed at day 7. **A.** Epidermal thickness. **B.** Eosinophil and mast cell numbers in dermis at the site of sensitization. Data are expressed as mean ± SEM (n=6-10 animals per group). * Statistically different from PBS-treated mice (P < 0.05); $ Statistically different from CX3CR1^{+/gfp} donor cells (P < 0.05).

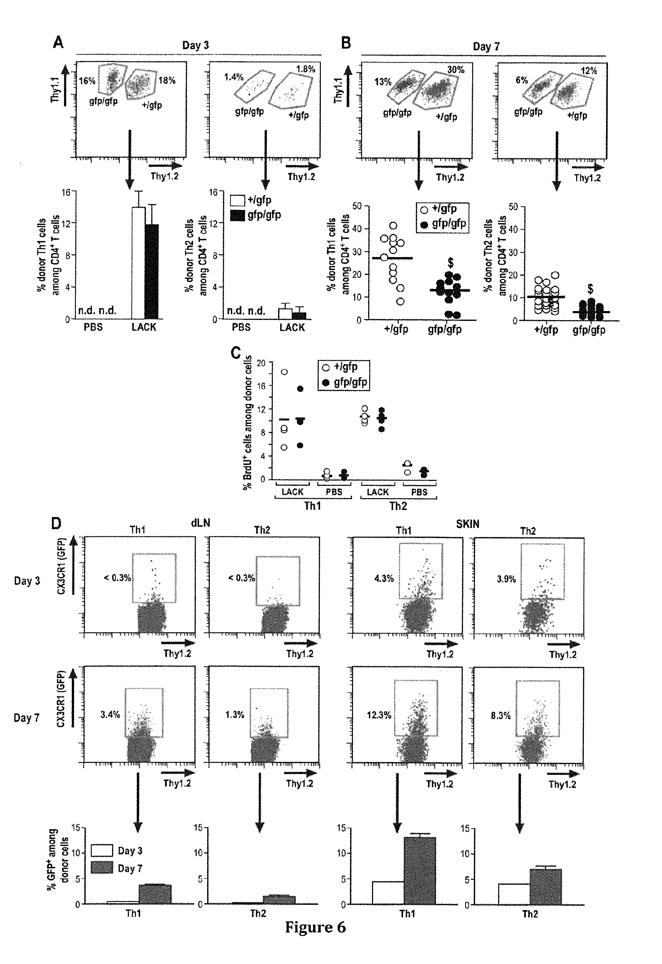

**Figure 6****.** CX3CR1 provides a selective advantage to effector CD4⁺ T cells. Equal numbers of LACK-specific CX3CR1^{gfp/gfp} (black histograms or symbols) and CX3CR1^{+/gfp} (white histograms or symbols) Th2 or Th1 cells were co-injected into WT mice at day 0. Recipients were sensitized with LACK or PBS at day 1 and day 4 and analyzed at day 3 (**A,C,D**) or day 7 (**B,D**). **A.** Donor cells were analyzed in skin by flow cytometry. Data show representative flow cytometry profiles (upper panels). Data show mean frequencies ± SEM of donor Th1 (bottom left panel) or Th2 (bottom right panel) cells among the CD4⁺ T cell population. One representative experiment out of two is shown. (*n*=6 mice per group). **B.** Donor cells were analyzed in skin by flow cytometry (upper panels). Data show donor cell frequency of Th1 (bottom left panel) or Th2 (bottom right panel) in individual mice with bars indicating the mean from three experiments (*n*=16 mice per group). **C.** 18 h before sacrifice, recipient mice were injected with BrdU, and donor cells were analyzed by flow cytometry after staining with anti-BrdU, anti-Thy1.1, anti-Thy1.2, anti-CD4, or anti-CD45 antibodies. Data show cell frequency of donor Th1 or Th2 cells in individual mice with bars indicating the mean in LACK-sensitized (*n*=4 mice) and PBS-sensitized mice (*n*=2-3 mice). (One representative experiment out of two). **D.** Donor cells were analyzed in draining LN and skin by flow cytometry for GFP expression at days 3 (white bars) and 7 (grey bars). Data show representative flow cytométrie profiles after aggregating files from individual mice (upper panels) (*n*=6 mice per group). One experiment out of three is shown. Data show mean frequencies ± SEM of GFP+ cells among Th1 (left panels) or Th2 (right panels) donor cells (*n*=12 mice per group at day 3 and *n*=18 mice at day 7).

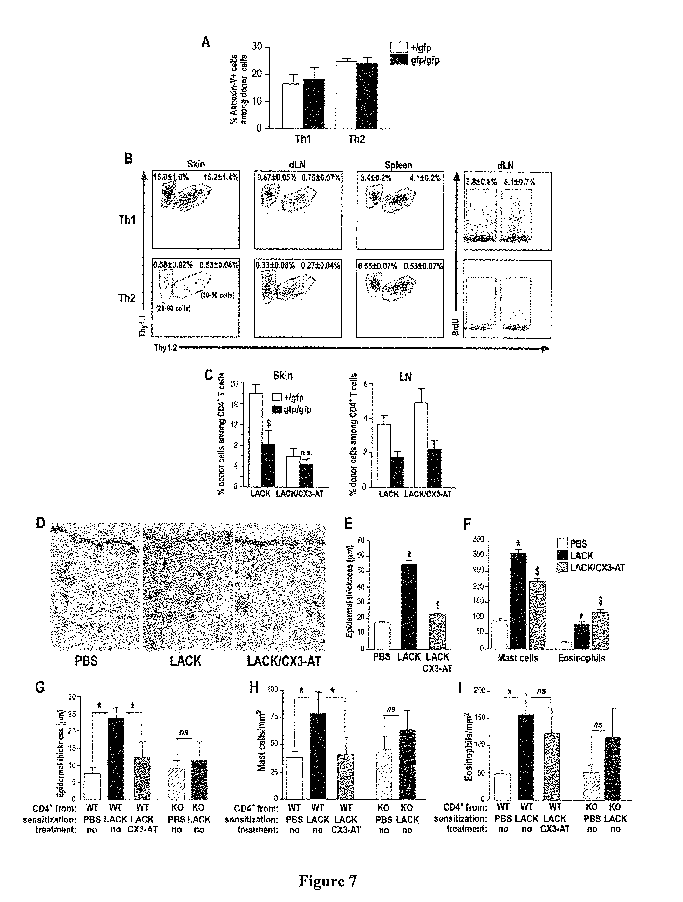

**Figure 7****.** CX3CR1 is required for T cell retention in chronically inflamed skin. **A.** Equal numbers of LACK-specific CX3CR1^{gfp/gfp} (black histograms) and CX3CR1^{+/gfp} (white histograms) Th2 or Th1 cells were co-injected into WT recipient mice at day 0. Recipients were sensitized with LACK at day 1 and day 4 and analyzed at day 3 and day 7. Donor cells were analyzed by flow cytometry 3 days later in inguinal draining LN after staining with antibodies against CD45, CD4, Thy1.1, Thy1.2, and annexin-V and 7-AAD. Data show mean frequencies of annexin-V⁺ cells among donor cells ± SEM. **B.** AD was induced in WT mice as described in the legend of Fig.1 and equal numbers of LACK-specific CX3CR1^{gfp/gfp} (left population) and CX3CR1^{+/gfp} (right population) Th1 (upper panels) or Th2 (lower panels) cells were co-injected at day 41. Donor cells were analyzed in skin, draining LN and spleen by flow cytometry. Data show representative flow cytometry profiles. Numbers indicate frequencies ± SEM of donor Th1 (upper panels) or Th2 (bottom panels) cells among the CD4⁺ T cell population. One representative experiment out of two is shown (*n*=12 mice per group). 18 h before sacrifice, recipient mice were injected with BrdU and donor cells were analyzed by flow cytometry after staining with anti-BrdU, -Thy1.1, -Thy1.2, -CD4 or -CD45 antibodies. Data show representative cytometry profiles and numbers indicate the mean (*n*=12 mice) (One representative experiment out of two). **C.** Equal numbers of LACK-specific CX3CR1^{gfp/gfp} (black histograms) and CX3CR1^{+/gfp} (white histograms) effector cells were co-injected into WT recipient mice at day 0. Recipients were sensitized with LACK at day 1 and day 4. At day 4, some recipient mice topically received LACK together with CX3-AT. Three days later, the frequencies of donor cells were analyzed in skin and draining LN by flow cytometry. Data show mean of donor cell frequency of individual mice in one representative experiment out of two (*n*=5 mice per group). $ Statistically different from CX3CR1^{+/gfp} donor cells (P < 0.05). **D-F.** AD was induced as described in the legend of Fig.1. During the last round of sensitization, some mice also received CX3-AT (50 µg/mouse) through the patch together with LACK as indicated on the figure. **D.** May Grünwald Giemsa staining of skin sections (original magnification X10). **E, F.** Epidermal thickness. **G, I.** Mast cell and eosinophil numbers. Data show mean of cell numbers in one representative experiment out of two (*n*=6 mice per group). * Statistically different from PBS-treated mice (P < 0.05); $ statistically different from vehicle-treated mice (P < 0.05). **G-I.** CX3CR1^{gfp/gfp} mice were injected with polyclonal naive WT or CX3CR1^{gfp/gfp} (KO) CD4⁺ T cells 24h prior to AD induction. During the last round of sensitization, mice also received CX3-AT (50 µg/mouse) through the patch together with LACK as indicated on the figure. **G.** Epidermal thickness. **H**, **I.** Mast cell and eosinophil numbers. Data show mean of cell numbers in one representative experiment (*n*=5 mice per group). * P < 0.05.

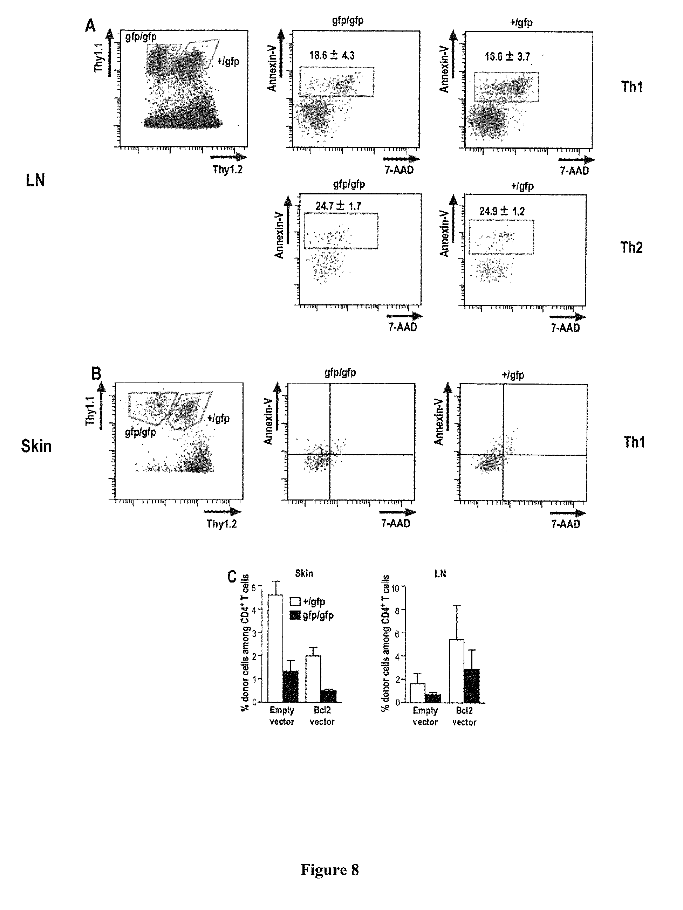

**Figure 8****.** The absence of CX3CR1 does not induce preferential T cell death. CX3CR1^{+/gfp} Thy1.1^{+/-} and CX3CR1^{gfp/gfp} Thy1.1^{+/+} WT15 Th1 or CX3CR1^{+/gfp} Thy1.1^{+/-} and CX3CR1^{gfp/gfp} Thy1.1^{+/+} WT15 Th2 cells were co-injected i.v. into WT Thy1.1^{-/-} Thy1.2^{+/+} mice one day before LACK epicutaneous sensitization. Donor cells were analyzed by flow cytometry 3 days later in inguinal draining LN (**A**) or in skin (**B**) after staining with antibodies against CD45, CD4, Thy1.1, Thy1.2, and annexin-V and 7-AAD. **C.** CX3CR1^{+/gfp} (white histograms) and CX3CR1^{gfp/gfp} (black histograms) Th2 cells were infected with a Bcl2 or an empty retroviral vector. Equal numbers of transduced cells were co-injected into recipients that were further sensitized with one round of LACK sensitization. Data show the mean of donor cell frequency in skin (left panel) and inguinal draining LN (right panel) of individual mice in one representative experiment out of two (*n*=5 mice per group).

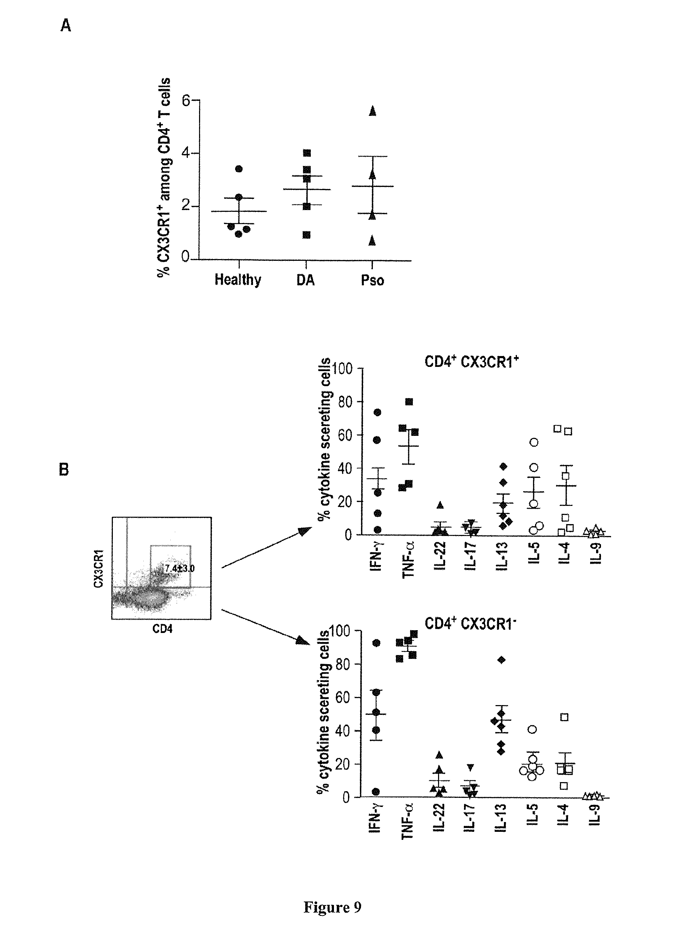

**Figure 9****.** CX3CR1 is expressed by skin-infiltrating CD4⁺ T cells in AD patients. **A.** CX3CR1 expression by circulating CD4⁺ T cells. PBMC were from patients with AD or from healthy donors were analyzed by flow cytometry for CX3CR1 expression. Data show frequency of CX3CR1⁺ cells among CD4⁺ T cells in individual donor. **B.** Cells from skin biopsies were characterized by surface and intracellular staining by flow cytometry. Left panel show a representative FACS profile. Numbers indicate the mean ± SEM of the frequency of CX3CR1⁺ among CD4⁺ T cells from n=6 patients. Right panels show frequencies of cytokine-secreting CX3CR1⁺ and CX3CR1⁻ CD4⁺ T cells for each patient.

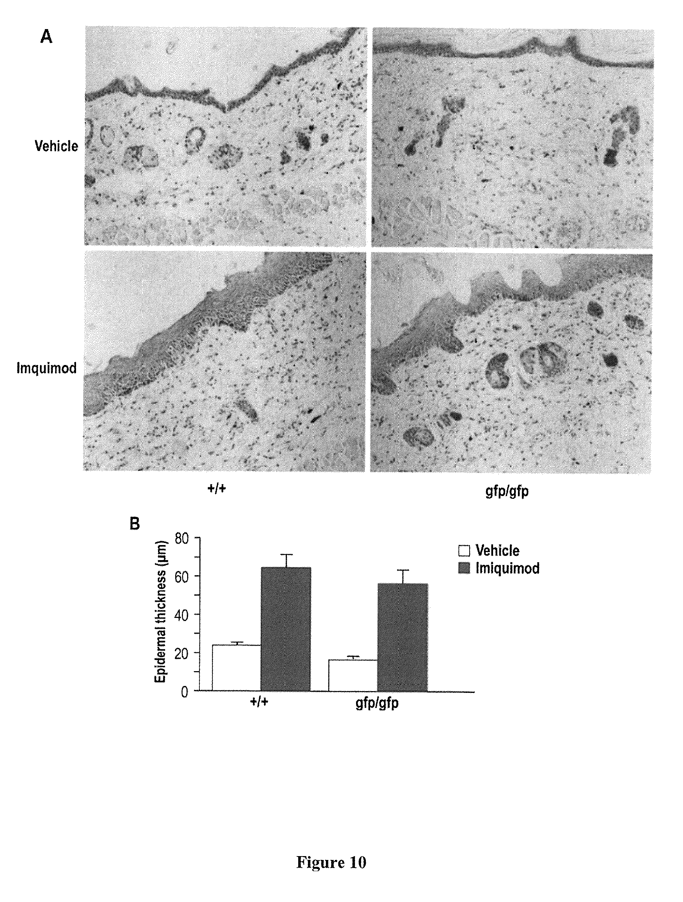

**Figure 10****.** The absence of CX3CR1 does affect development of psoriasis. Psoriasis was induced on abdominal skin in CX3CR1^{+/+} and CX3CR1^{gfp/gfp} mice by topical application of 62 mg Imiquimod or vehicle for 6 days and sacrificed 24h later. Skin samples were collected at the site of sensitization. **A.** May Grünwald Giemsa staining of skin sections (original magnification X10 for the main panels and X63 for the inset). **B.** Epidermal thickness.

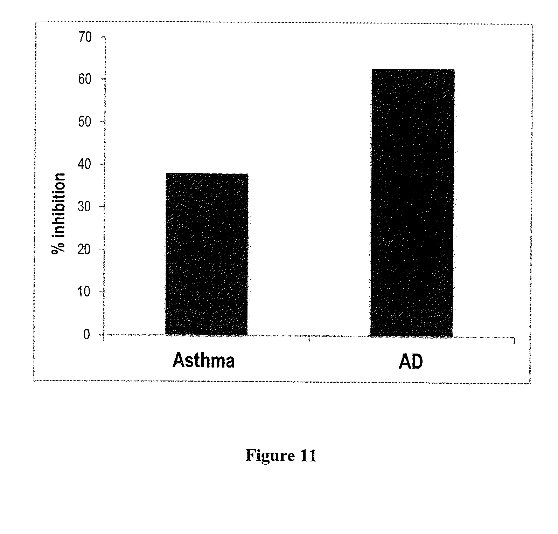

**Figure 11****.** Percentage of inhibition of AHR and epidermal thickness in a mouse model of allergic asthma and AD, respectively upon local treatment with CX3CR1 peptide antagonist. The percentage was calculated as follows: [1- (Mean resistance value measured in treated mice (n=8) / Mean resistance value obtained in untreated mice)] x 100 for asthma; [1-(Mean epidermal thickness value measured in treated mice (n=6) / Mean epidermal thickness value obtained in untreated mice)] x 100 for AD.

### EXAMPLE 1: CX3CL1 (FRACTALKINE) AND ITS RECEPTOR CX3CR1 REGULATE ATOPIC DERMATITIS BY CONTROLLING EFFECTOR T CELL RETENTION IN INFLAMED SKIN

### MATERIALS AND METHODS

### Animals

CX3CR1^{gfp/gfp}, CX3CR1^{+/gfp}, CX3CR1^{gfp/gfp} and CX3CR1^{+/gfp} Thy1.1^{+/-} WT15 TCR transgenic mice were generated in a Balb/c ByJ background as previously described (Mionnet *et al.,* 2010). Eight- to 12-week-old female mice were used for all experiments. Animals were housed within the specific pathogen-free facility from the Institut Pasteur de Lille (France) or from the Institut de Pharmacologie Moléculaire et Cellulaire in Sophia-Antipolis (France). Experiments were performed after approval by the Ethics Committee for Animal Experimentation from Lille (France) and Nice (France).

### Experimental atopic dermatitis

As previously described, AD was induced by epicutaneous sensitization (Staumont-Salle *et al.*, 2008) with LACK antigen (GenBank Accession No. AAA97577.1 referred to as SEQ ID NO: 36; Mougneau *et al.*, 1995). Briefly, patches soaked with 25 µL of 0.2% LACK solution in PBS or with vehicle were applied on abdominal skin 24 hours after shaving and were left on for three 1-week periods (with patch renewal at midweek), with a 2-week interval between applications. At the time of the last patch removal (day 49), animals were challenged for 20 minutes by means of aerosol nebulization with LACK (0.2% in PBS) by using an ultrasonic nebulizer (Systam, Villeneuve sur Lot, France) and serum was collected. On the next day, airway hyperresponsiveness (AHR) to increasing concentrations of methacholine was measured by means of invasive plethysmography using a FlexiVent and expressed by dynamic lung resistance (SCIREQ) (Kanda *et al.,* 2009). Animals were sacrificed by cervical dislocation. Bronchoalveolar lavage fluid (BALF) was collected and analyzed on cytospin preparations after RAL 555 staining. Skin and inguinal lymph nodes were collected for histological analyses and real-time PCR after RNA extraction. LACK was produced and detoxified using Endotrap columns (Profos). Endotoxin levels assessed by LAL assay (Pierce) were below 5 ng/mg of protein.

### Treatment with a CX3CL1 antagonist (CX3-AT) in a model of AD

CX3-AT blocking reagent (SEQ ID NO: 31) was prepared as described by Mionnet *et al.* (2010) and administered by weekly intraperitoneal injections (50 µg/mouse) either during the 3 sensitization periods (prophylactic protocol) or only during the last week of sensitization (therapeutic protocol) and animals were analyzed as described above. Alternatively, CX3-AT was applied topically (50 µg/mouse) by patch together with the antigen during the last 3 days of a single round of sensitization or during the third and last week-long round of sensitization.

### Histology and immunohistochemistry

Tissue biopsy specimens were processed as previously described (Staumont-Salle *et al.,* 2008). Briefly, samples were fixed in ImmunoHistoFix (Interstiles, Brussels, Belgium) and embedded in ImmunoHistoWax (Interstiles) at 37°C. Five µm sections were stained with May Grünwald Giemsa for measurement of epidermal thickness and eosinophil and mast cells counts (Staumont-Salle *et al.,* 2008) by using a microscope with Arcturus XT software. Epidermal thickness was determined at 250-fold magnification; an average of 10 measures was calculated for each sample. Eosinophils and mast cells were enumerated by examining 20 random fields at 400-fold magnification; cell frequency was converted to cells per square millimeter and results were expressed as mean ± SD. For immunohistochemical analysis, sections embedded in ImmunoHistoWax were immunostained with anti-I-Ad/I-Ed (MHC II) mAb (clone M5/114, rat IgG2b, BD Biosciences) and cryopreserved sections were stained with anti-CD4 mAb (clone RM4-5, rat IgG2a, BD Biosciences) as previously described (Angeli *et al.,* 2004). For each section, 10 random fields were examined at x400.

### Immunoglobulin concentrations

Immunoglobulin (IgE, IgG₁ and IgG₂ₐ) concentrations in serum were measured as previously described by ELISA (Staumont-Salle *et al.*, 2008). Two-fold serial dilutions were prepared for each serum (starting dilution 1:25 for IgE, 1:5000 for IgG¹, 1:1000 for IgG^{2a}). Antibody titers were calculated as the dilution corresponding to twice the mean absorbance value obtained for non-sensitized mouse sera.

### Quantitative RT-PCR

mRNA steady-state levels were analyzed by quantitative RT-PCR as previously described (Staumont-Salle *et al.,* 2008). Oligonucleotide primers specific for mouse cytokines, chemokines, and their receptors and nuclear transcription factors are listed in Table 1 below.

**Table 1: Sequences of primers used for RT-PCR**

| **Gene** | **Primer** | **Sequence 5' to 3'** | **SEQ ID NO.** |
|---|---|---|---|
| Mouse GAPDH | Forward | CGT CCC GTA GAC AAA ATG GT | 1 |
| | Reverse | AGG TCA ATG AAG GGG TCG TT | 2 |
| Mouse IL-4 | Forward | GGT CTC AAC CCC CAG CTA GTT G | 3 |
| | Reverse | ATG GCG TCC CTT CTC CTG TG | 4 |
| Mouse IL-5 | Forward | TGA CCG CCA AAA AGA GAA GTG T | 5 |
| | Reverse | TTG CCC ATT CTG TAC TCA TCA CA | 6 |
| Mouse IL-12p35 | Forward | CCA AAC CAG CAC ATT GAA GA | 7 |
| | Reverse | AGC TCC CTC TTG TTG TGG AA | 8 |
| Mouse IL-13 | Forward | TTG CAT GGC CTC TGT AAC CG | 9 |
| | Reverse | CGT GGC GAA ACA GTT GCT TT | 10 |
| Mouse IL-6 | Forward | CCA CGG CCT TCC CTA CTT CA | 11 |
| | Reverse | CCA CGA TTT CCC AGA GAA CAT G | 12 |
| Mouse IFN-γ | Forward | GCT TTG CAG CTC TTC CTC AT | 13 |
| | Reverse | CCA GTT CCT CCA GAT ATC CAA G | 14 |
| Mouse TNF-α | Forward | GTC TAC TGA ACT TCG GGG TGA | 15 |
| | Reverse | CTC CTC CAC TTG GTG GTT TG | 16 |
| Mouse CCR4 | Forward | CGG CAT TGC TTC ATA GAC TG | 17 |
| | Reverse | GTT TCA TCC TGG GTG GTG TC | 18 |
| Mouse CCR5 | Forward | ATC CGT TCC CCC TAC AAG AG | 19 |
| | Reverse | GCA GGG TGC TGA CAT ACC AT | 20 |
| Mouse CXCR3 | Forward | AGC CAA GCC ATG TAC CTT GA | 21 |
| | Reverse | GAG TCA GAG AAG TCG CTC TC | 22 |
| Mouse T-bet | Forward | CCA ACA ATG TGA CCC AGA TG | 23 |
| | Reverse | AGC TGA GTG ATC TCT GCG TTC | 24 |
| Mouse GATA-3 | Forward | GCC TGC GGA CTC TAC CAT AA | 25 |
| | Reverse | CAT TAG CGT TCC TCC TCC AG | 26 |
| Mouse STAT-4 | Forward | CCT GGG TGG ACC AAT CTG AA | 27 |
| | Reverse | CTC GCA GGA TGT CAG CGA A | 28 |
| Mouse STAT-6 | Forward | CAT GAA CAA CAC GGT GCC C | 29 |
| | Reverse | GGT GAC AGA CTC TGT GCC CTT C | 30 |

Primers were used for amplification in triplicate assays. PCR amplification of GAPDH was performed to control for sample loading and to allow normalization between samples. Relative gene expression was calculated with the 2^{-ΔΔCT} method (Livak and Schmittgen, 2001). Results were expressed as the mean fold induction compared with the mean expression level in PBS-treated WT mice.

### Murine cell purification and in vivo assays

For the antigen-presenting capacity assay, splenic CD4⁺ T cells were purified from CX3CR1^{+/+} WT15 Thy1.1^{+/+} Rag-2^{-/-} TCR transgenic mice, encoding a LACK specific TCR, by positive selection using anti-CD4 beads (MACS, Miltenyi) (purity >95%), stained with CFSE (Sigma) and further injected (2 x 10⁶ cells) via the lateral tail vein into CX3CR1^{gfp/gfp} and CX3CR1^{+/gfp} recipient mice, 1 day before epicutaneous LACK sensitization. Proliferation of donor cells in the inguinal LN was assessed by flow cytometry 5 days later.

For analysis of CX3CR1^{+/+} and CX3CR1^{gfp/gfp} T cell proliferation, splenic CD4⁺ T cells were purified from CX3CR1^{gfp/gfp} Thy1.1^{+/+} and CX3CR1^{+/gfp} Thy1.1^{+/-} WT15 donor mice, stained with CFSE, and co-injected (2 x 10⁶ of each population) into Thy1.1^{-/} CX3CR1^{+/+} wild-type mice, one day before epicutaneous LACK sensitization. Proliferation was assessed in the inguinal LN 4 days later by flow cytometry.

For preparation of Th1 and Th2 cells, CD4⁺ T cells from TCR transgenic mice were purified by negative selection and 1.5 x 10⁶ cells were incubated for 3 days with 0.75 x 10⁶ T cell depleted splenocytes in complete RPMI with 50 nM LACK₁₅₆₋₁₇₃ peptide and (i) 10 ng/ml r-IL-4 and 10 µg /ml antibody to IFNγ (R4-6A2) for Th2 cells or (ii) 10 ng/ml r-IL-12 and 10 µg/ml antibody to IL-4 (11B11) for Th1 cells. In some experiments, Thy1.1^{-/-} Thy1.2^{+/+} WT mice received either CX3CR1^{gfp/gfp} or CX3CR1^{+/gfp} LACK-specific Th1 or Th2 cells (2 x 10⁶ cells per mouse) by intravenous injection and then underwent one single round of LACK epicutaneous sensitization. Animals were sacrificed at day 7 and skin samples were collected for histological analysis of AD characteristics as previously described (Staumont-Salle *et al.,* 2008). In other experiments, the same number of both CX3CR1^{gfp/gfp} and CX3CR1^{+/gfp} LACK-specific Th1 or CX3CR1^{gfp/gfp} and CX3CR1^{+/gfp} LACK-specific Th2 cells were co-injected in Thy1.1^{-/-} Thy1.2^{+/+} WT mice mice (1.5 x 10⁶ of each genotype per mouse), one day before LACK epicutaneous sensitization. When indicated, recipient mice treated as described above, were injected i.p. with BrdU (BD Biosciences) (200 µg) for the last 18 hrs. BrdU incorporation of CX3CR1 -proficient and -deficient Th1 and Th2 donor cells was studied by flow cytometry in inguinal LN. Survival of Th1 and Th2 donor cells was analyzed by annexin-V and 7-aminoactinomycin D (7-AAD) staining in both skin and inguinal LN.

In reconstitution experiments, 5 x 10⁶ naïve CX3CR1^{gfp/gfp} or CX3CR1^{+/gfp} CD4⁺ T cells were adoptively transferred into CX3CR1^{gfp/gfp} mice 24h prior to induction of AD.

### Human T cells

PBMC and skin samples were obtained from patients with AD from the Department of Dermatology or from healthy donors from the Department of Plastic Surgery (Claude Huriez Hospital, Lille, France). This study was approved by the Ethical Committee from the University Hospital of Lille. All subjects provided written informed consent. Patients with AD were selected according to the Hanifin and Rajka criteria (Hanifin and Rajka, 1980). PBMC were obtained after elimination of granulocytes on a Ficoll gradient. Punch skin biopsies (diameter 5 mm) were cultured in complete RPMI 1640 supplemented with 100 U/ml penicillin, 100 µg/ml streptomycin (all Invitrogen), 5% human serum (Sigma) and 20U IL-2/ml (Novartis). After 10-13 days, emigrating cells were collected and characterized by surface and intracellular staining by flow cytometry. Surface and intracellular cytokine staining was performed using the Cytofix/Cytoperm kit (BD Biosciences) according to the manufacturer's instructions. The following fluorochrome-conjugated antibodies were used: anti-IFN-γ-V450 (B27), anti-TNFAlexa fluor 700 (Mab11), anti-IL-4-PerCP-Cy5.5 (8D4-8), anti-IL17A-PE (N49-653), anti-IL-5-APC (TRFK5), anti-IL-9 PerCP-Cy5.5 (MH9A3), anti-CD4-APC-Cy7 (RPA-T4), anti-IL-13-Horizon-V450 (JES10-5A2) (all BD Bioscience), anti-IL4-PE (3010.211), (BD FastImmune), anti-IL22-APC (142928) (R&D), CX3CR1-FITC (2A9-1) Biolegend.

### Retroviral transduction

The human Bcl2 cDNA was cloned into the mouse bi-cistronic retroviral expression vector MIGR. Th2 differentiated cells were incubated with viral supernatant containing 5 µg/ml Polybrene (Sigma) and spun at 32°C for 8 h as described (Mionnet et al., 2010). Viral supernatant was replaced with fresh medium. GFP-expressing T cells were sorted 3 days later.

### Psoriasis induction

Psoriasis was induced in CX3CR1^{gfp/gfp} and CX3CR1^{+/+} mice by topical application of 62 mg Imiquimod for 6 days (van der Fits *et al.,* 2009). Lanette cream (Fagron, Waregem, Belgium) was used as control. After sacrifice, skin was analyzed for epidermal thickening and cellular recruitment as described for atopic dermatitis.

### Statistical analysis

Statistical significance was determined by Student T test, except for plethysmographic data, for which ANOVA for repeated measures was used. Graphpad and STATview softwares were used, respectively. Results were expressed as means ± SEM. *A P* value <0.05 was considered significant.

### RESULTS

### Upon skin sensitization, CX3CR1-deficient mice develop neither AD nor subsequent lung inflammation.

To assess the contribution of CX3CR1 to AD development, it was used a previously described model of AD based on repeated epicutaneous sensitizations (Spergel *et al.,* 1998) with *Leishmania major activated C Kinase* (LACK) as antigen and compared the response of CX3CR1-deficient (^{gfp/gfp}) mice, in which the CX3CR1 gene has been replaced by gfp (Jung *et al.,* 2000), to that from their proficient (^{+/+}) WT counterparts. Neither strain exhibited an inflammatory phenotype in the absence of LACK sensitization (Fig. 1A). Compared to vehicle (*i.e*. PBS)-sensitized CX3CR1^{+/+} mice, LACK-sensitized CX3CR1^{+/+} mice exhibited a significant skin inflammatory response, characterized by a 50% increase in epidermal thickening (Fig. 1B) associated with more pronounced hyperkeratosis, spongiosis and dermal cellular infiltrates including mast cells, eosinophils, MHC-II+ and CD4⁺ T cells (Fig. 1C) as well as increased skin and inguinal lymph node (LN) expression of inflammatory and Th1-and Th2-associated cytokines, chemokines and chemokine receptors (Fig. 2A). In sharp contrast, CX3CR1^{gfp/gfp} mice did not develop a skin inflammatory response upon LACK sensitization (Fig. 1A-B). Compared with PBS-sensitized CX3CR1^{gfp/gfp} mice, only MHC-II⁺ cell numbers were increased, but to a lesser extent than in LACK-sensitized CX3CR1^{+/+} animals (Fig. 1C). Furthermore, expression of Th1- and inflammatory response genes was also significantly decreased, while among Th2- response genes, only skin STAT-6 and lymph node GATA-3 expression were affected (Fig. 2A-B).

Humoral response was also altered in CX3CR1^{gfp/gfp} compared to CX3CR1^{+/+} mice, with decreased total Th2-associated IgE concentrations (but not IgG1 titers), as well as decreased Th1-associated antigen-specific IgG2a titers (Fig. 1D).

As in the human pathology, epicutaneous sensitization also induced lung inflammation and airway hyperreactivity (AHR) following a single antigenic airway challenge. Airway resistance upon LACK-sensitization was significantly lower in CX3CR1^{gfp/gfp} compared to CX3CR1^{+/+} animals (Fig. 1E). Furthermore, cellular inflammatory infiltrates in the bronchoalveolar fluid (BALF) of LACK-sensitized CX3CR1^{gfp/gfp} mice were decreased by 32% for macrophages, 70% for lymphocytes and eosinophils, and 40% for neutrophils compared to BALF from CX3CR1^{+/+} mice (Fig. 1F).

### A CX3CL1 antagonist strongly reduces features of AD.

To further confirm the key role of CX3CR1 in AD development, it was next investigated whether inhibition of CX3CL1/CX3CR1 interactions would inhibit the pathology in WT animals. It was investigated the efficacy of a CX3CR1 antagonist (CX3-AT), whose potency was already validated in an allergic asthma model (Mionnet *et al.,* 2010), using prophylactic or therapeutic administration protocols (Fig. 3A). Both administration schedules fully inhibited antigen-induced epidermal thickening (Fig. 3B), as well as dermal mast cell, eosinophil and CD4⁺ T cell infiltration (Fig. 3C). Upon LACK aerosol challenge, AHR and inflammatory cell infiltration in the airways were also significantly decreased upon both prophylactic and therapeutic treatments (Fig. 3D-E). Taken together, these results confirm the key role of CX3CR1/CX3CL1 in AD in non-genetically manipulated mice and further demonstrate that pharmacological inhibition of CX3CL1/CX3CR1 interactions abrogate skin and lung inflammation.

### CX3CR1-deficiency neither affects antigen presentation nor naïve T cell proliferation in vivo.

As CX3CR1 is expressed by various myeloïd cells, such as blood monocytes, dendritic cell (DC) progenitors, plasmacytoid DC, and macrophages (Bar-On *et al.,* 2010; Kim *et al.,* 2011; Zhang *et al.,* 2012), it was next assessed whether antigen presentation was affected in the absence of CX3CR1. To this aim, antigen-specific CD4⁺ T cells from WT15 TCR transgenic mice (Wang *et al.,* 2001), were labeled with CSFE and injected to both CX3CR1^{+/gfp} and CX3CR1_{gfp/gfp} mice that were further sensitized via epicutaneous LACK administration. Frequencies of divided antigen-specific CD4⁺ T cells in the draining LN were comparable in both CX3CR1^{+/gfp} and CX3CR1^{gfp/gfp} mice (Fig. 4A), suggesting that, upon epicutaneous sensitization, CX3CR1 deficiency does not affect antigen presentation. It was next investigated whether CX3CR1 deficiency affected T cell proliferation induced upon epicutaneous antigen sensitization. To address this issue, it was generated Thy1^{+/-} CX3CR1^{+/gfp} and Thy1^{+/+} CX3CR1^{gfp/gfp} LACK-specific TCR transgenic mice that respectively expressed the Thy1.1 and Thy1.2 antigens or the Thy1.1 antigen only. CD4⁺ T cells from both genotypes were prepared, stained with CSFE and co-injected into WT Thy1.1^{/-} Thy1.2^{+/+} mice. Upon epicutaneous sensitization with LACK, frequencies of dividing CX3CR1^{+/gfp} and CX3CR1^{gfp/gfp} WT15 cells were comparable (Fig. 4B). Altogether these results suggest that CX3CR1 deficiency does not alter naïve T cell proliferation.

### CX3CR1 expression regulates both Th1- and Th2- induced skin inflammation.

As both Th2 and Th1 cells are associated to the acute and chronic phases of AD, respectively (Spergel *et al.,* 1999), it was investigated whether CX3CR1 expression was required by Th1, Th2 cells or both to induce skin inflammation. LACK-specific CD4⁺ T cells were prepared from either Thy1^{+/-} CX3CR1+/gfp or Thy1+/+ CX3CR1^{gfp/gfp} WT15 mice, differentiated *in vitro* into Th1 or Th2 cells and injected into WT mice that were further sensitized to LACK. While both CX3CR1-proficient Th1 and Th2 cells alone were able to induce a 4 to 5-fold epidermal thickening upon a single round of epicutaneous antigen exposure, CX3CR1-deficient Th2 cells only induced a less than 2-fold increase in epidermal thickness and injection of CX3CR1-deficient Th1 cells barely led to epidermal thickening compared to PBS-treated animals (Fig. 5A). Likewise, dermal mast cell infiltration was also induced by both CX3CR1-proficient Th1 and Th2 cells, but not upon injection of CX3CR1-deficient effector T cells (Fig. 5B). Eosinophil infiltration was only induced upon transfer of CX3CR1-proficient Th2 cells and strongly reduced when CX3CR1-deficient Th2 cells were injected (Fig. 5B). Taken together, these results suggest that CX3CR1 expression by Th2- and Th1-cells regulates the key features of AD.

### CX3CR1 expression confers a selective advantage to skin-infiltrating T cells.

To decipher the mechanisms accounting for the role of CX3CR1 expression by T helper cells in skin inflammation, it was monitored the recruitment and proliferative capacities of both CX3CR1-proficient and CX3CR1-deficient LACK-specific Th1 and Th2 cells upon co-injection into WT mice that were exposed to LACK and further fed with BrdU. Three days following antigen exposure, while Th1 and Th2 donor cells of both genotypes had not yet incorporated BrdU (data not shown), CX3CR1-proficient and CX3CR1-deficient LACK-specific donor cells were detected at the same frequency in skin, suggesting that early migration of effector T cells into the skin did not require CX3CR1 (Fig. 6A). It is worth noting that antigen-induced recruitment of LACK-specific Th1 cells was more pronounced than recruitment of Th2 cells as early as 3 days after antigen exposure. In sharp contrast, frequencies of CX3CR1-proficient donor cells outnumbered CX3CR1-deficient cells on day 7 (Fig. 6B). However, donor cells of both genotypes proliferated at the same rate (Fig. 6C). Therefore, as observed for naïve T cells, CX3CR1 deficiency neither affects the early recruitment, nor the proliferation of Th1 - and Th2-effector cells.

It was next monitored CX3CR1 expression using GFP as a surrogate marker for CX3CR1 expression (Geissmann *et al.,* 2003; Jung *et al.,* 2000). Upon differentiation, antigen-specific effector cells remained GFP- as previously described (Mionnet *et al.,* 2010). At day 3 after initial sensitization, less than 0.3% of antigen-specific effector cells expressed CX3CR1 (*i.e.,* GFP) in draining LN, and around 4% of these cells expressed CX3CR1 (i.e., GFP) in skin (Fig. 6D). At day 7, while the frequencies of CX3CR1-expressing Th1 and Th2 cells in the draining LN slightly increased to 3.6 and 1.4 %, respectively, their frequencies in skin steadily increased to 12.3 and 8.3 %, respectively (Fig. 6D). These latter results suggest that CX3CR1 expression is likely to be induced in skin and involved at a late time.

### CX3CR1-deficiency impairs effector T cell retention into inflamed skin.

The higher frequency of CX3CR1-proficient CD4⁺ effector cells in skin could be explained by several hypotheses: the preferential late recruitment of CX3CR1⁺ effector T cells into the skin, their prolonged survival, or their selective advantage for residence in the inflamed skin. As CX3CR1 is involved in effector T cell survival in allergic lung inflammation (Mionnet *et al.,* 2010), and as the role of CX3CR1/CX3CL1 in microglial cell (Meucci , 1998) and monocyte survival (Landsman *et al.,* 2009) has also been reported, it was next assessed whether this was also the case in inflamed skin. WT mice were co-injected with CX3CR1-proficient and -deficient antigen-specific Th1- or Th2- donor cells, sensitized by epicutaneous antigen administration and the frequencies of apoptotic and/or necrotic donor cells were measured. Similar frequencies of annexin-V⁺ and/or 7-AAD⁺ CX3CR1^{+/gfp} and CX3CR1^{gfp/gfp} donor cells were found in both skin and draining LN (Fig. 7A, and 8A-B), suggesting that CX3CR1 is not involved in T cell survival. However, as Th2 donor cells were difficult to monitor in skin due to their low frequencies, additional experiments were performed to confirm these data. CX3CR1^{+/gfp} and CX3CR1^{gfp/gfp} antigen-specific Th2 cells were transduced with a retroviral construct leading to the expression of anti-apoptotic BCL-2 (an empty vector was used as control) and transferred into WT recipients that were sensitized by LACK. While overexpression of BCL-2 led to a decrease, and respectively an increase in the recovery of transduced cells in the skin and draining LN, it did not affect the ratio between CX3CR1-proficient and -deficient Th2 cells within these tissues, ruling out a role of CX3CR1 in T cell survival (Fig. 8C).

It was next compared the migration of CX3CR1-proficient and -deficient effector T cells into the inflamed skin and draining LN of WT mice with established AD. Recipient mice were submitted to two rounds of epicutaneous sensitization prior to the injection of CX3CR1-proficient and -deficient Th1 or Th2 cells the day before the third and last antigen application. Three days later, while BrdU incorporation was very low, frequencies of donor cells of both genotypes were similar (Fig. 7B), demonstrating that CX3CR1 is not required for the late migration of effector T cells into the inflamed skin. To investigate whether CX3CR1 was required for T cell retention into the inflamed skin, mice were co-injected with CX3CR1-proficient and -deficient LACK-specific effector T cells, challenged for 4 days with LACK, and further challenged with the antigen while topically treated with CX3-AT for 3 days. Upon topical CX3-AT treatment, frequency of skin CX3CR1-proficient T cells decreased to the level of co-injected CX3CR1-deficient cells, while their frequency increased in draining LN (Fig. 7C), demonstrating that blocking CX3CR1-CX3CL1 interactions *in situ* prevents CX3CR1⁺ T cell retention.

Next, to further corroborate these findings with endogenously generated T cells, WT mice were treated topically with CX3-AT for the last 3 days of the third and last round of epicutaneous sensitization. CX3-AT strongly decreased epidermal thickening, mildly inhibited mast cell infiltration and did not significantly affect eosinophil infiltration (Fig. 7D-F).

Finally, to further confirm that the effect of CX3CR1 in AD was due to its expression on T cells, it was adoptively transferred CX3CR1-proficient or -deficient naïve T cells into CX3CR1-deficient animals and induced AD. After 7 weeks, animals reconstituted with CX3CR1-proficient T cells developed a pathology (epidermal thickening, mast cell and eosinophil infiltration) that was comparable to that of CX3CR1-proficient animals. Reconstitution with CX3CR1-deficient T cells did not induce symptoms of AD (Fig. 7G-I). Furthermore, topical application of CX3-AT during the last 3 days of sensitization, to CX3CR1-deficient mice reconstituted with CX3CR1-proficient T cells, the only cells expressing CX3CR1 in this experimental setting, exerted similar effect than on (non-reconstituted) CX3CR1-proficient animals (Fig. 7G-I).

Altogether, these data demonstrate that blocking interactions between CX3CR1 and *CX3CL1 in situ* prevents disease symptoms by impairing effector T cell retention in inflamed skin.

### Human Th2- and Th1- CX3CR1⁺ cells infiltrate AD skin lesions.

It was investigated whether CX3CR1⁺ CD4⁺ T cells could be present in AD patients. In agreement with the absence of CX3CR1 expression on circulating mouse CD4⁺ T cells, even upon antigenic sensitization, it was detected a very low expression of circulating human CD4⁺ T in AD patients, and also in healthy patients (Fig. 9A). About 7% of skin isolated CD4⁺ T cells expressed CX3CR1, in keeping with the findings in the murine model of AD. As CX3CR1⁻ cells, CX3CR1⁺ CD4⁺ T cells display a very heterogeneous cytokine profile with the Th1 and Th2 subsets being the most represented as compared to Th17 (Fig. 9B).

### CONCLUSION

It was demonstrated that in an AD model based on epicutaneous antigen sensitization, CX3CR1 deficiency prevented skin inflammatory response and severely reduced the pulmonary symptoms as well as humoral responses. This defect was solely due to CX3CR1 expression by CD4⁺ T cells as demonstrated by reconstitution experiments in which transfer of WT CD4⁺ T cells in CX3CR1-deficient mice restored skin disease that could be further blocked upon CX3CR1 antagonist treatment. Paradoxically for an allergic disease and unlike asthma, the most pronounced inhibitory effects were observed on the Th1-associated rather than Th2-associated response (Fig. 2 and 5A). These findings further underline the major role exerted by CX3CR1 on Th1 cells, prominently associated to the chronic phase of the disease, which adds to its key effect on Th2 cells in allergic asthma (Mionnet *et al.,* 2010).

CX3CL1 and expression of its unique receptor, CX3CR1 on T cells play a crucial role in the development of AD by retaining effector T cells in the inflamed skin. Indeed, neither proliferation, early or late migration, nor survival were affected by CX3CR1 deficiency on CD4⁺ effector T cells, while blocking CX3CR1/CX3CL1 interactions *in situ* prevented WT allergen-specific T cell accumulation in skin and induced their accumulation in draining LN (Fig. 7C). Interestingly, upon such treatment, while allergen-specific T cells were more abundant in the periphery, this later also prevented the development of pulmonary symptoms observed upon intranasal antigen challenge.

Chemokines known so far to be involved in skin diseases contribute to the pathology by inducing migration of inflammatory cells towards the skin and not by facilitating their retention in the inflammatory sites. Indeed, Islam *et al.* demonstrated that CCL8 is involved in skin homing of CCR8-expressing Th2 cells in the same animal model of AD (Islam *et al.,* 2011). Likewise, Homey et al showed that intradermal CCL27 injection attracted lymphocytes *in vivo* and, conversely, neutralization of interactions between CCL27 and its receptor CCR10 impaired lymphocyte recruitment to the skin leading to the suppression of DNFB (dinitrofluorobenzene)-induced skin inflammation in a murine model of contact hypersensitivity (Homey *et al.*, 2002). While the membrane form of CX3CL1 had been previously shown to mediate adhesion of CX3CR1-expressing leukocytes *in vitro*, the results reported herein demonstrate such a role *in vivo.* The role of CX3CR1 seems to be restricted to AD as CX3CR1-deficient and -proficient mice displayed similar epidermal thickening and inflammation in a well-established experimental model mimicking human psoriasis by repeated applications of Imiquimod (a TLR7/8 agonist) (van der Fits *et al.*, 2009) (Fig. 10).

The identification of a new mode of action for a chemokine and the full inhibition of AD by a CX3CR1 antagonist further demonstrates that the CX3CR1/CX3CL1 axis represents a new therapeutic target in atopic dermatitis.

### EXAMPLE 2: COMPARISON OF TREATMENT EFFICACY BETWEEN ALLERGIC ASTHMA AND ATOPIC DERMATITIS

The treatment efficacy with the peptide CX3-AT has been compared between allergic asthma described by Mionnet *et al.* (2010) and atopic dermatitis described above. The results are shown in Fig. 11 representing the percentage of inhibition of clinical symptoms upon local administration of CX3CR1 peptide antagonist.

Compared to untreated asthmatic mice, asthmatic mice treated intranasally with CX3-AT exhibited decreased AHR in response to inhaled methacholine (from Fig. 2c. of Mionnet *et al.,* 2010). The CX3-AT treatment provided 38% inhibition (see Fig. 11).

Similarly, topically sensitized mice that were further treated with CX3-AT developed less severe cutaneous lesion than their untreated counterparts. The epidermal thickness was indeed decreased by 63% upon local CX3-AT treatment (see Fig. 7E and 11).

Therefore, the efficacy of local treatment with the peptide CX3C-AT is better on atopic dermatitis than in allergic asthma.

### REFERENCES

Angeli, V., et al., 2004. J Immunol 172:3822-3829*.*
Berard, F., et al., 2003. Eur J Dermatol 13:324-330.
Bar-On, L., et al., 2010. Proc Natl Acad Sci USA 107:14745-14750.
Depner, M., et al., 2007. Int Arch Allergy Immunol 144:91-94.
Echigo, T., et al., 2004. JAllergy Clin Immunol 113:940-948.
Fong, A.M., et al., J Exp Med 188:1413-1419.
Fraticelli, P., et al., 2001. J Clin Invest 107:1173-1181.
Geissmann, F., et al., 2003. J Exp Med 198:623-634.
Grewe, M., et al., 1998. Immunol Today 19:359-361.
Guttman-Yassky, E., et al., 2011a. J Allergy Clin Immunol 127:1110-1118.
Guttman-Yassky, E., et al., 2011b. J Allergy Clin Immunol 127:1420-1432.
Hanifin, J.M., and G. Rajka. 1980. Acta Derm Venerol Suppl 92:44-47.
Homey, B., et al., 2002. Nat Med 8:157-165.
Imai, T., et al., 1997. Cell 91:521-530.
Inoue, A., et al., 2005. Arthritis Rheum 52:1522-1533.
Islam, S.A., et al., 2011. Nat Immunol 12:167-177.
Julia, V. 2012. Allergy 67:1106-1110.
Jung, S., et al., 2000. Mol Cell Biol 20:4106-4114.
Kagami, S., et al., 2003. Clin Exp Immunol 134:309-313.
Kanda, A., et al., 2009. J Allergy Clin Immunol 124:573-582, 582 e571-579.
Kim, K.W., et al., 2011. Blood 118:e156-167.
Landsman, L., et al., 2009. Blood 113:963-972.
Leung, D.Y., et al., 2004. J Clin Invest 113:651-657.
Liu, X., et al., 2011. Mol Biol Rep. 38:481-8
Livak, K.J., and T.D. Schmittgen. 2001. Methods 25:402-408.
McComb, J.G., et al., 2008. Am JPathol 173:949-961*.*
Meucci, O., et al., 1998. Proc Natl Acad Sci US A 95:14500-14505.
Mionnet, C., et al., 2010. Nat Med 16:1305-1312.
Mougneau, E., et al., 1995. Science 268:563-566.
Nakayama, T., et al., 2010. J Immunol 185:6472-6479.
Nanki, T., et al., 2002. Arthritics Rheum 46:2878-2883.
Owczarek, W., et al., 2011. Cytokine 50:181-185.
Reiss, Y., et al., 2001. J Exp Med 194:1541-1547.
Ren, T., et al., 2007., Biochem Biophys Res Commun 364:978-84.
Ruitenberg, M.J., et al., 2008. J Neuroimmunol 205:80-85.
Schall, T.J., and A.E. Proudfoot. 2011. Nat Rev Immunol 11:355-363.
Spergel, J.M., et al., 1998. J Clin Invest 101:1614-1622.
Spergel, J.M., et al., 1999. J Clin Invert 103:1103-1111.
Staumont-Salle, D., et al., 2008. J Allergy Clin Immunol 121:962-968 e966.
Tremblay, K., et al., 2006. Genes Immun 7:632-639.
van der Fits, L., et al., 2009. J Immunol 182:5836-5845.
Wang, Q., et al., 2001. J Immunol 167:4311-4320.
Zhang, X., S. et al., 2012. Blood 119:3975-3986.

## Claims

1. An inhibitor of the expression of fractalkine (CX3CL1) or an inhibitor of the expression of the receptor of fractalkine (CX3CR1) or an inhibitor of the CX3CL1/CX3CR1 interaction for use in the treatment of atopic dermatitis.

2. An inhibitor of the CX3CL1/CX3CR1 interaction for use according to claim 1, **characterized in that** it is selected from the group consisting of a peptide, an organic molecule, a peptidomimetic, an antibody or fragment thereof, and an aptamer.

3. An inhibitor of the CX3CL1/CX3CR1 interaction for use according to claim 1 or claim 2, **characterized in that** it is a competitive antagonist of CX3CR1.

4. An inhibitor of the CX3CL1/CX3CR1 interaction for use according to claim 3, **characterized in that** it a peptide CX3-AT of sequence SEQ ID NO. 32, 33, 34 or 35.

5. An inhibitor of the expression of CX3CL1 or of CX3CR1 for use according to claim 1, **characterized in that** it is selected from the group consisting of a antisense oligonucleotide, an interfering RNA and a ribozyme, targeting the gene encoding CX3CL1 or the gene encoding CX3CR1 respectively.

6. An inhibitor of the CX3CL1/CX3CR1 interaction for use according to claim 3, **characterized in that** the peptide CX3-AT is administered topically.

7. An inhibitor of the expression of CX3CL1 or of CX3CR1 or an inhibitor of the CX3CL1/CX3CR1 interaction for use according to any one of claims 1 to 6, **characterized in that** the subject to treat is a human.

8. An inhibitor of the expression of CX3CL1 or of CX3CR1 or an inhibitor of the CX3CL1/CX3CR1 interaction for use according to any one of claims 1 to 6, **characterized in that** the subject to treat is a dog.

9. An inhibitor of the CX3CL1/CX3CR1 interaction, **characterized in that** it is a peptide selected from the group consisting of SEQ ID NO: 34 and SEQ ID NO: 35.


SEQUENCE LISTING

<110>  UNIVERSITE DE NICE SOPHIA ANTIPOLIS
       CENTRE NATIONAL DE LA RECHERCHE SCIENTIFIQUE

<120>  INHIBITION OF THE FRACTALKINE-RECEPTOR INTERACTION FOR THE
       TREATMENT OF ATOPIC DERMATITIS

<130>  XRN/clg/F2245-7/EP

<160>  36

<170>  PatentIn version 3.5

<210>  1
<211>  20
<212>  DNA
<213>  Artificial Sequence

<220>
<223>  Primer

<400>  1
cgtcccgtag acaaaatggt                                                  20


<210>  2
<211>  20
<212>  DNA
<213>  Artificial Sequence

<220>
<223>  Primer

<400>  2
aggtcaatga aggggtcgtt                                                  20


<210>  3
<211>  22
<212>  DNA
<213>  Artificial Sequence

<220>
<223>  Primer

<400>  3
ggtctcaacc cccagctagt tg                                               22


<210>  4
<211>  20
<212>  DNA
<213>  Artificial Sequence

<220>
<223>  Primer

<400>  4
atggcgtccc ttctcctgtg                                                  20


<210>  5

<211>  22
<212>  DNA
<213>  Artificial Sequence

<220>
<223>  Primer

<400>  5
tgaccgccaa aaagagaagt gt                                                22


<210>  6
<211>  23
<212>  DNA
<213>  Artificial Sequence

<220>
<223>  Primer

<400>  6
ttgcccattc tgtactcatc aca                                               23


<210>  7
<211>  20
<212>  DNA
<213>  Artificial Sequence

<220>
<223>  Primer

<400>  7
ccaaaccagc acattgaaga                                                   20


<210>  8
<211>  20
<212>  DNA
<213>  Artificial Sequence

<220>
<223>  Primer

<400>  8
agctccctct tgttgtggaa                                                   20


<210>  9
<211>  20
<212>  DNA
<213>  Artificial Sequence

<220>
<223>  Primer

<400>  9
ttgcatggcc tctgtaaccg                                                   20


<210>  10
<211>  20
<212>  DNA
<213>  Artificial Sequence

<220>
<223>  Primer

<400>  10
cgtggcgaaa cagttgcttt                                                    20


<210>  11
<211>  20
<212>  DNA
<213>  Artificial Sequence

<220>
<223>  Primer

<400>  11
ccacggcctt ccctacttca                                                    20


<210>  12
<211>  22
<212>  DNA
<213>  Artificial Sequence

<220>
<223>  Primer

<400>  12
ccacgatttc ccagagaaca tg                                                 22


<210>  13
<211>  20
<212>  DNA
<213>  Artificial Sequence

<220>
<223>  Primer

<400>  13
gctttgcagc tcttcctcat                                                    20


<210>  14
<211>  22
<212>  DNA
<213>  Artificial Sequence

<220>
<223>  Primer

<400>  14
ccagttcctc cagatatcca ag                                                 22


<210>  15
<211>  21
<212>  DNA
<213>  Artificial Sequence

<220>
<223>  Primer

<400>  15
gtctactgaa cttcggggtg a                                                21


<210>  16
<211>  20
<212>  DNA
<213>  Artificial Sequence

<220>
<223>  Primer

<400>  16
ctcctccact tggtggtttg                                                  20


<210>  17
<211>  20
<212>  DNA
<213>  Artificial Sequence

<220>
<223>  Primer

<400>  17
cggcattgct tcatagactg                                                  20


<210>  18
<211>  20
<212>  DNA
<213>  Artificial Sequence

<220>
<223>  Primer

<400>  18
gtttcatcct gggtggtgtc                                                  20


<210>  19
<211>  20
<212>  DNA
<213>  Artificial Sequence

<220>
<223>  Primer

<400>  19
atccgttccc cctacaagag                                                  20


<210>  20
<211>  20
<212>  DNA
<213>  Artificial Sequence

<220>
<223>  Primer

<400>  20
gcagggtgct gacataccat                                                  20

<210>  21
<211>  20
<212>  DNA
<213>  Artificial Sequence

<220>
<223>  Primer

<400>  21
agccaagcca tgtaccttga                                                    20


<210>  22
<211>  20
<212>  DNA
<213>  Artificial Sequence

<220>
<223>  Primer

<400>  22
gagtcagaga agtcgctctc                                                    20


<210>  23
<211>  20
<212>  DNA
<213>  Artificial Sequence

<220>
<223>  Primer

<400>  23
ccaacaatgt gacccagatg                                                    20


<210>  24
<211>  21
<212>  DNA
<213>  Artificial Sequence

<220>
<223>  Primer

<400>  24
agctgagtga tctctgcgtt c                                                  21


<210>  25
<211>  20
<212>  DNA
<213>  Artificial Sequence

<220>
<223>  Primer

<400>  25
gcctgcggac tctaccataa                                                    20


<210>  26

<211>  20
<212>  DNA
<213>  Artificial Sequence

<220>
<223>  Primer

<400>  26
cattagcgtt cctcctccag                                                   20


<210>  27
<211>  20
<212>  DNA
<213>  Artificial Sequence

<220>
<223>  Primer

<400>  27
cctgggtgga ccaatctgaa                                                   20


<210>  28
<211>  19
<212>  DNA
<213>  Artificial Sequence

<220>
<223>  Primer

<400>  28
ctcgcaggat gtcagcgaa                                                    19


<210>  29
<211>  19
<212>  DNA
<213>  Artificial Sequence

<220>
<223>  Primer

<400>  29
catgaacaac acggtgccc                                                    19


<210>  30
<211>  22
<212>  DNA
<213>  Artificial Sequence

<220>
<223>  Primer

<400>  30
ggtgacagac tctgtgccct tc                                                22


<210>  31
<211>  95
<212>  PRT
<213>  Artificial Sequence

<220>
<223>  Peptide antagonist

<220>
<221>  BINDING
<222>  (1)..(73)
<223>  CX3-AT

<220>
<221>  DOMAIN
<222>  (74)..(89)
<223>  Bir A sequence

<220>
<221>  DOMAIN
<222>  (90)..(95)
<223>  Histidine tag

<400>  31

Met Thr Lys Cys Glu Ile Met Cys Asp Lys Met Thr Ser Arg Ile Pro
1               5                   10                  15

Val Ala Leu Leu Ile Arg Tyr Gln Leu Asn Gln Glu Ser Cys Gly Lys
            20                  25                  30

Arg Ala Ile Val Leu Glu Thr Thr Gln His Arg Arg Phe Cys Ala Asp
        35                  40                  45

Pro Lys Glu Lys Trp Val Gln Asp Ala Met Lys His Leu Asp His Gln
    50                  55                  60

Ala Ala Ala Leu Thr Lys Asn Gly Gly Ser Leu His His Ile Leu Asp
65                  70                  75                  80

Ala Gln Lys Met Val Trp Asn His Arg His His His His His His
                85                  90                  95

<210>  32
<211>  73
<212>  PRT
<213>  Artificial Sequence

<220>
<223>  Peptide antagonist

<400>  32

Met Thr Lys Cys Glu Ile Met Cys Asp Lys Met Thr Ser Arg Ile Pro
1               5                   10                  15

Val Ala Leu Leu Ile Arg Tyr Gln Leu Asn Gln Glu Ser Cys Gly Lys
            20                  25                  30

Arg Ala Ile Val Leu Glu Thr Thr Gln His Arg Arg Phe Cys Ala Asp
        35                  40                  45

Pro Lys Glu Lys Trp Val Gln Asp Ala Met Lys His Leu Asp His Gln
    50                  55                  60

Ala Ala Ala Leu Thr Lys Asn Gly Gly
65                  70

<210>  33
<211>  74
<212>  PRT
<213>  Artificial Sequence

<220>
<223>  Peptide antagonist

<400>  33

Met Thr Lys Cys Glu Ile Met Cys Asp Lys Met Thr Ser Arg Ile Pro
1               5                   10                  15

Val Ala Leu Leu Ile Arg Tyr Gln Leu Asn Gln Glu Ser Cys Gly Lys
            20                  25                  30

Arg Ala Ile Val Leu Glu Thr Thr Gln His Arg Arg Phe Cys Ala Asp
        35                  40                  45

Pro Lys Glu Lys Trp Val Gln Asp Ala Met Lys His Leu Asp His Gln
    50                  55                  60

Ala Ala Ala Leu Thr Lys Asn Gly Gly Lys
65                  70

<210>  34
<211>  73
<212>  PRT
<213>  Artificial Sequence

<220>
<223>  Peptide antagonist

<400>  34

Val Thr Lys Cys Asn Ile Thr Cys Ser Lys Met Thr Ser Lys Ile Pro
1               5                   10                  15

Val Ala Leu Leu Ile His Tyr Gln Gln Asn Gln Ala Ser Cys Gly Lys
            20                  25                  30

Arg Ala Ile Ile Leu Glu Thr Arg Gln His Arg Leu Phe Cys Ala Asp
        35                  40                  45

Pro Lys Glu Gln Trp Val Lys Asp Ala Met Gln His Leu Asp Arg Gln
    50                  55                  60

Ala Ala Ala Leu Thr Arg Asn Gly Gly
65                  70

<210>  35
<211>  77
<212>  PRT
<213>  Artificial Sequence

<220>
<223>  Peptide antagonist

<400>  35

Gln His Leu Gly Val Lys Lys Cys Asn Val Thr Cys His Lys Met Thr
1               5                   10                  15

Ser Glu Ile Pro Val Ala Leu Leu Val His Tyr Gln Arg Asn Gln Glu
            20                  25                  30

Ser Cys Gly Lys Pro Ala Ile Ile Leu Lys Thr Lys Gln Asn Arg Ile
        35                  40                  45

Phe Cys Ala Asp Pro Lys Glu Arg Trp Val Gln Lys Ala Ile Ala His
    50                  55                  60

Leu Glu His Gln Ile Ala Val Thr Ile Arg Asn Gly Gly
65                  70                  75

<210>  36
<211>  312
<212>  PRT
<213>  Leishmania major

<400>  36

Met Asn Tyr Glu Gly His Leu Lys Gly His Arg Gly Trp Val Thr Ser
1               5                   10                  15

Leu Ala Cys Pro Gln Gln Ala Gly Ser Tyr Ile Lys Val Val Ser Thr
            20                  25                  30

Ser Arg Asp Gly Thr Val Ile Ser Trp Lys Ala Asn Pro Asp Arg His
        35                  40                  45

Ser Val Asp Ser Asp Tyr Gly Leu Pro Asn His Arg Leu Glu Gly His
    50                  55                  60

Thr Gly Phe Val Ser Cys Val Ser Leu Ala His Ala Thr Asp Tyr Ala
65                  70                  75                  80

Leu Thr Ala Ser Trp Asp Arg Ser Ile Arg Met Trp Asp Leu Arg Asn
                85                  90                  95

Gly Gln Cys Gln Arg Lys Phe Leu Lys His Thr Lys Asp Val Leu Ala
            100                 105                 110

Val Ala Phe Ser Pro Asp Asp Arg Leu Ile Val Ser Ala Gly Arg Asp
        115                 120                 125

Asn Val Ile Arg Val Trp Asn Val Ala Gly Glu Cys Met His Glu Phe
    130                 135                 140

Leu Arg Asp Gly His Glu Asp Trp Val Ser Ser Ile Cys Phe Ser Pro
145                 150                 155                 160

Ser Leu Glu His Pro Ile Val Val Ser Gly Ser Trp Asp Asn Thr Ile
                165                 170                 175

Lys Val Trp Asn Val Asn Gly Gly Lys Cys Glu Arg Thr Leu Lys Gly
            180                 185                 190

His Ser Asn Tyr Val Ser Thr Val Thr Val Ser Pro Asp Gly Ser Leu
        195                 200                 205

Cys Ala Ser Gly Gly Lys Asp Gly Ala Ala Leu Leu Trp Asp Leu Ser
    210                 215                 220

Thr Gly Glu Gln Leu Phe Lys Ile Asn Val Glu Ser Pro Ile Asn Gln
225                 230                 235                 240

Ile Gly Phe Ser Pro Asn Arg Phe Trp Met Cys Val Ala Thr Glu Arg
                245                 250                 255

Ser Leu Ser Val Tyr Asp Leu Glu Ser Lys Ala Val Ile Ala Glu Leu
            260                 265                 270

Thr Pro Asp Gly Ala Lys Pro Ser Glu Cys Ile Ser Ile Ala Trp Ser
        275                 280                 285

Ala Asp Gly Asn Thr Leu Tyr Ser Gly His Lys Asp Asn Leu Ile Arg
    290                 295                 300

Val Trp Ser Ile Ser Asp Ala Glu
305                 310